# EUROPÄISCHE PATENTSCHRIFT

(11) **EP 1 429 669 B1**
(45) Veröffentlichungstag und Bekanntmachung des Hinweises auf die Patenterteilung: **15.04.2009**
(21) Anmeldenummer: 02751174.0
(22) Anmeldetag: 09.08.2002
(51) Int. Cl.: A61B 17/34, A61M 25/06

(54) **VERWEILKATHETERANORDNUNG**
INDWELLING CATHETER ARRANGEMENT
ENSEMBLE CATHETER A DEMEURE

(30) Priorität: 24.09.2001 DE 10146853
(43) Veröffentlichungstag der Anmeldung: 23.06.2004
(73) Patentinhaber: RADIMED Schmerztherapie und Kathetertechnik GmbH, 44805 Bochum (DE)
(72) Erfinder: MARIANOWICZ, Martin, 81679 München (DE); KLEIN, Ralf, 42555 Velbert (DE); HENKE, Jan, 45701 Herten (DE); FEIT, Herbert, 63607 Wächtersbach (DE); HAAS, Michael, 63619 Bad Orb (DE); KRUSE, Reinhard, 63571 Gelnhausen (DE)
(74) Vertreter: Stoffregen, Hans-Herbert
(86) Internationale Anmeldenummer: PCT/EP2002/008961
(87) Internationale Veröffentlichungsnummer: WO 2003/028564

(56) Entgegenhaltungen:
- EP-A- 0 331 318
- EP-A- 0 357 999
- US-A- 4 874 376
- US-A- 5 106 376
- US-A- 5 735 813
- US-A- 5 976 110

## Beschreibung

Die Erfindung bezieht sich auf eine Venveilkantheteranordnung umfassend eine Kanüle, deren proximales Ende von einer Aufnahme ausgeht und dessen distales Ende eine Öffnung, insbesondere einen Tuohyschliff mit seitlicher Öffnung aufweist, sowie einen durch die Aufnahme und durch die Öffnung führbaren flexiblen Katheter, in dem seinerseits beim Legen des Katheters ein Führungselement verläuft, wobei die Verweilkatheteranordnung eine Halterung aufweist und der Katheter mit seinem proximalen Ende verschiebbar in der Halterung verläuft.

Über entsprechende Verweilkatheteranordnungen können im Epiduralraum eines Patienten über einen gewünschten Zeitraum Medikamente verabreicht werden. Hierzu verbleibt der Katheter in dem Epiduralraum in dem Bereich, in dem eine medikamenlöse Behandlung erfolgen soll. Zum Legen des Katheters wird zunächst die Kanüle über den Hiatus Sacralis oder im Bereich einer Wirbeletage in den Epiduralraum eingeführt, um sodann über die Kanüle den Katheter zu plazieren. Damit dieser in hinreichendem Umfang geführt und beim Verschieben in dem Epiduralraum eine notwendise Steifigkeit aufweist, befindet sich in dem Katheter ein Führumgselement wie Führungsdraht, der nach der Positionierung des Katheters entfernt wird. Hierdurch besteht die Gefahr, dass durch das Herausziehen eine Lageveränderung des Katheters erfolgt.

Beim Positionieren des Katheters sollte dieser in seinem distalen Bereich eine gewünschte Flexibilität aufweisen. Hierzu sieht ein von der Epimed International Inc. vertriehene Verweilkatheteranordnung einen aus einer Spiralfeder hergestellten Katheter vor, der ummantelt ist. Die Spiralfeder ist am distalen Ende im Abstand zur Spitze gestreckt, um somit einerseits eine gewünschte Flexibilität zu erzielen und andererseits durch den Katheter geführte Flüssigkeit austreten zu lassen. Ein beim Legen des Katheters in diesem verlaufelder Führungsdraht wird nach ordnungsgemäßer Positionierung entfernt.

Nachleilig bei einem entsprechenden Spiralfederkatheter ist es, dass beim Herausziehen des Katheters aus dem Epiduralraum bei weiterhin plazierter Kanüle die Spiralfeder an der einen Schliff aufweisenden Öffnung des Katheters abscheren oder hängen bleiben kann, so dass ein Auseinanderziehen der Spiralfeder erfolgt. Dies wiederum Führt zu einem aufwendigen und gegebenenfalls zu gesundheitlichen Schäden führendes Entfernen der "langgestreckten Spiralfeder" aus den Körper.

Aus der US-A-5,976,110 ist eine Verweilkatheteranordnung bekannt, die eine Kanüle mit einer distalen Tuohyschlifföffnung und einen durch die Kanüle führbaren flexiblen Katheter aufweist.

Der vorliegenden Erfindung liegt das Problem zu Grunde, eine Verweilkatheteranordnung der eingangs genannten Art so weiterzubilden, dass der Katheter im distalen Bereich eine gewünschte Flexibilität aufweist, wobei gleichzeitig sichergestellt ist, dass bei positioniertem Katheter eine ungewollte Lageveränderung unterbleibt. Auch soll ein problemloses Entfernen des Katheters nach Beendigung der insbesondere medikamentösen Behandlung möglich sein. Gegebenenfalls soll auch die Möglichkeit geschaffen werden, eine Stimulation im Bereich des distalen Endes des Katheters zu bewirken.

Erfindungsgemäß wird das Problem im Wesentlichen dadurch gelöst, dass das Führungselement in der Halterung fixierbar ist und dass das nach dem Legen des Katheters in diesem verbleibende Führungselement in der Halterung relativ zu dem Katheter in definierter Position fixiert ist.

Dabei sollte das Führungselement wie ein Draht oder ein Rohr während des Legens des Katheters zu diesem in einer ersten Position und bei gelegtem Katheter zu diesem in einer zweiten Position relativ fixiert sein. Somit besteht die Möglichkeit, dass der Katheter in seinem distalen Ende beim Legen eine andere Flexibilität aufweist als beim Verabreichen eines Medikamentes. Unabhängig hiervon erfolgt eine unerwünschte Lageveränderung des Katheters nicht, da während der gesamten Behandlung das Führungselement in dem Katheter verbleibt.

Insbesondere sollte das Führungselement grundsätzlich innerhalb des Katheters verlaufen. Zur Erzielung einer Elektrostimulation kann jedoch auch vorgesehen sein, dass das Führungselement das distale Ende des Katheters durchsetzt und diesen überragt.

Bei dem Katheter selbst handelt es sich vorzugsweise um einen Schlauch aus Kunststoff wie aus Polyamid. Polyurethan. Silikon oder entsprechenden Materialien, so dass dieser problemlos über die Kanüle eingeführt bzw. nach Beendigung der Behandlung aus dieser entfernt werden kann. Dabei kann der Katheter auch endseitig geschlossen sein. Zum Austritt von medikamentösen Mitteln sind sodann im distalen Bereich Öffnungen wie Löcher vorhanden. Entsprechende bzw. eine größer Anzahl von Öffnungen sind dann vorgesehen, wenn über das Führungselement eine Elektrostimulation erfolgen soll, ohne dass das Führungselement den Katheter distal überragt.

In Weiterbildung der Erfindung ist vorgesehen, dass die Verweilkatheteranordnung eine insbesondere bereichsweise rohrformige Halterung aufweist, in der einerseits das Führungselement fixiert ist und andererseits der Katheter mit seinem proximalen Ende verläuft, das in der Halterung verschiebbar ist. Nach Verstellen des Katheters entlang des Führungselementes wird ersterer vorzugsweise durch ein Klemmelement festgelegt. Hierdurch erfolgt das relative Fixieren zwischen Katheter und Führungselement.

Insbesondere wird der Katheter über einen Tuohy-Burst-Adapter oder einen entsprechenden Adapter, der das erforderliche Klemmen ermöglicht, fixiert, wobei der Katheter über ein Gummielement durch Quetschen festgelegt werden kann.

Die Halterung weist einen Fluidanschluss auf, über den das zu applizierende Medikament zugeführt wird, damit dieses über die Halterung und den Katheter an den gewünschten Ort in dem Epiduralraum strömen kann. Der Fluidanschluss bietet die Möglichkeit, einen standardisierten Filter aufzuschieben. Im distalen Bereich des Katheters verlaufende Fluid - austrittsöffnung bzw. -Öffnungen können in der Katheterwandung verlaufen oder durch einen endseitig offenen Katheter gebildet werden.

In besonders hervorzuhebender Ausgestaltung der Erfindung weist die Halterung eine Y- oder V-förmige Geometrie mit einem entlang einer Gerade verlaufenden ersten Schenkel und einem zu diesem geneigt verlaufenden und in diesen übergehenden zweiten Schenkel auf, wobei in dem ersten Schenkel das Fuhrungselement fixiert und in diesem das proximale Ende des Katheters verschiebbar angeordnet sind. Dabei kann das Führungselement im in Bezug auf die Kanüle entfernt liegenden stirnseitigen Randbereich des ersten Schenkels fixiert sein.

Nach einem weiteren Vorschlag kann das Führungselement in einem proximalen Bereich der Halterung durch Klemmen, insbesondere durch Klemmen eines gebogenen Endes des Führungselementes fixiert sein.

Alternativ kann die Halterung ein Rohrelement sein, in dem das Führungselement fixiert ist. Dabei sollte das Führungselement von einem zylinderischen in dem Führungselement positionierten Halteelement ausgehen, das seinerseits zumindest einen Fluiddurchlass aufweist. Der Fluiddurchlass weist insbesondere mehrere im Schnitt auf einem Ring angeordnete das Führungselement konzentrisch umgebende Durchlässe auf.

Das Führungselement besteht vorzugsweise aus einer Form-Gedächtnis-Legierung wie Nickel-Titan-Legierung oder enthält dieses.

Ist das Führungselement als Draht ausgebildet, so sollte dessen Durchmesser zwischen 0,30 mm und 0,55 mm, wobei der Katheter einen Außendurchmesser von 0,8 mm bis 2.3 mm aufweisen sollte.

Auch besteht die Möglichkeit, das Führungselement als Rohr auszubilden. Dabei muss der Außendurchmesser auf das Lumen des Katheters derart abgestimmt sein dass das Führungselement verschiebbar ist und das Fluid durch das Lumen des Führungselementes an die Katheterspitze getangt.

Nach einer besonders hervorzuhebenden Ausgestaltung der Erfindung kann mittels der Verweilkatheteranordnung auch eine Stimulation wie Rückenmark-Stimulation dadurch erfolgen, das das aus elektrisch leitendem Material wie insbesondere Memory-Material bestehende Führungselement mit einem Pol einer Spannungsquelle verbunden wird. um sodann im distalen Bereich des Katheters, in dem das Endes des Führungselementes bei gelegte Katheter verlauft, die gewünschte Stimulation zu bewirken. Mit anderen Worten wird durch das Führungselement nicht nur gezielt die Steifigkeit des Katheters eingestellt. sondern zusätzlich übt das Führungselement die Funktion einer monopolaren Stimulationselektrode aus.

Um die elektrische Stimulation zu ermöglichen, durchsetzt das Führungselement entweder den Katheter distal oder beim Verlauf innerhalb des Katheters weist dieser im distalen Bereich mehrere Öffnungen auf, um so einen elektrisch leitenden Kontakt bzw. eine elektrisch leitende Verbindung mit einem Gewebe bzw. einer Gewebeflüssigkeit zu erreichen.

Bei geschlossenem Katheter sind insbesondere acht bis zwölf öffnungen vorgesehen, um im erforderlichen Umfang den erforderlichen Stromfluss zwischen Führungselement und Gewebe sicherzustellen.

Insbesondere ist vorgesehen, dass das Fuhrungselement in einer Halterung mit vorzugsweise V- oder Y-Geometrie fixiert ist, dass die Halterung einen ersten geradlinig verlaufenden Schenkel aufweist, entlang dem das Führungselement und der dieses umgebende und zu diesem verschiebbare Katheter abschnittsweise oder in dessen Richtung verläuft, und dass die Halterung einen geneigt zu dem ersten Schenkel verlaufenden zweiten Schenkel umfasst, in dem das Führungselement fixiert ist. Dabei kann das Führungselement einen in der Halterung flüssigkeitsdicht eingesetzten Stopfen unverrückbar durchsetzen und außerhalb der Halterung mit einem elektrischen Kontakt verbunden sein. Nach - einem alternativen Vorschlag ist vorgesehen, dass das Führungselement in der Halterung über einen eine Öffnung des zweiten Schenkels der Halterung verschließende Element wie Stopfen fixiert ist, der seinerseits flüssigkeitsdicht die Öffnung z. B. durch Einkleben verschließt.

Ein weiterer Vorschlag sieht vor, dass Verbindung zwischen proximalem Bereich des Führungselementes und einem elektrischen Leiter mit Kunststoff umspritzt ist, wobei dieser eine Stopfenform erfährt, der flüssigkeitsdicht in eine Öffnung der Halterung einsetzbar ist.

Auch kann das Führungselement von einer Metallbuchse ausgehen, die ihrerseits flüssigkeitsdicht in einer Öffnung der Halterung eingesetzt ist.

Weitere Einzelheiten, Vorteile und Merkmale der Erfindung ergeben sich nicht nur aus den Ansprüchen, den diesen zu entnehmenden Merkmalen - für sich und/oder in Kombination sondern auch aus der nachfolgenden Beschreibung von der Zeichnung zu entnehmenden bevorzugten Ausführungsbeispielen.

### Es zeigen:

- Fig. 1: eine erste Ausführungsform einer Venveilkatheteranordnung im Zustand einer Benutzungsmöglichkeit,
- Fig. 2: eine vergrößerte Darstellung einer der Fig. 1 zu entnehmenden Halterung für ein Führungselement,
- Fig. 3: eine verkleinerte Darstellung der Halterung gemäß Fig. 2.
- Fig. 4: ein Detail der Halterung gemäß Fig. 2 und 3,
- Fig. 5: Einzelteile der Verweilkatheteranordnung gemäß Fig. 1,
- Fig. 6: eine zweite Ausführungsform einer Verweilkatheteranordnung,
- Fig. 7: eine der der Fig. 6 zu entnehmende Halterung für ein Führungselement.
- Fig. 8: ein Detail der Halterung gemäß Fig. 7.
- Fig. 9: verschiedene Positionen eines Katheters zu einem Führungselement,
- Fig. 10: eine Ausführungsform der Fixierung des Führungselementes.
- Fig. 11: eine weitere Ausführungsform zur Fixierung des Führungselemetes.
- Fig. 12: eine erste Ausführungsform eines in einer Halterung fixierten Führungselementes mit elektrischen Anschluss.
- Fig. 13: eine zweite Ausführungsform eines in einer Halterung fixierten Führungselementes mit elektrischem Anschluss,
- Fig. 14: eine dritte Ausführungsform eines in einer Halterung fixierten Führungselementes mit elektrischem Anschluss,
- Fig. 15: in vergrößerter Darstellung distaler Bereich eines Katheters mit diesen durchsetzendem Führungselement und
- Fig. 16: eine vergrößerte Darstellung einer weiteren Ausführungsform eines Katheters im distalen Bereich mit innerhalb von diesem verlaufendem Führungselement.

Den Fig., in denen grundsätzlich gleiche Elemente dieselben Bezugszeichen aufweisen, sind Ausführungsformen von Verweilkatheteranordnungen 10 bzw. 100 zu entnehmen, mit denen im Epiduralraum eines Patienten in einem gewünschten Bereich ein Medikament appliziert werden kann. Dabei setzen sich die Verweilkatheteranordnungen 10, 100 aus an und für sich bekannten Elementen zusammen. So weist die Verweilkatheranordnung 10, 100 eine Kanüle 12 wie Spaltkanüle mit vorzugsweise Tuohy-Schliff mit seitlicher Öffnung 14 am distalen Ende auf. Das proximale Ende geht von einer Aufnahme 16 mit zentraler Bohrung aus, um die Kanüle 12 wahlweise mit einem Mandrin 18 zu verschließen oder die Aufnahme 18 mit einem Katheter 20 in Form eines Kunststoffschlauchs zum Beispiel aus Polyamid zu verbinden. Innerhalb des Schlauches 12 verläuft ein Führungselement 22 in Form eines Drahtes oder Rohres, durch die der Katheter 20 eine gewünschte Steifigkeit erfährt.

Um eine definierte relative Verschiebung zwischen dem Führungselement 22 und dem Katheter 20 zu ermöglichen, geht das Führungselement 22 von einer Halterung 24 bzw. 124 aus. die ihrerseits einen Anschluss 26, 126 aufweist, der mit einem Filierelement 28 verbindbar ist, über das das zu applizierende fluide Medikament strömt. Das Medikament wird über eine handelsübliche Spritze 31 und gegebenenfalls einen Verbindungsschlauch 33 appliziert.

Der Katheter 20 ist über die Aufnahme 16 der Kanüle 12 in den Epiduralraum einbringbar. Gegenüberliegendes Ende des Katheters 20 durchsetzt einen Tuohy-Burst-Adapter 30. der seinerseits mit der Halterung 24. 124 verbindbar ist.

Des Weiteren können die jeweiligen Anschlüsse bzw. Öffnungen über Verschlusskappen 32 in gewohnter Weise abgedichtet werden.

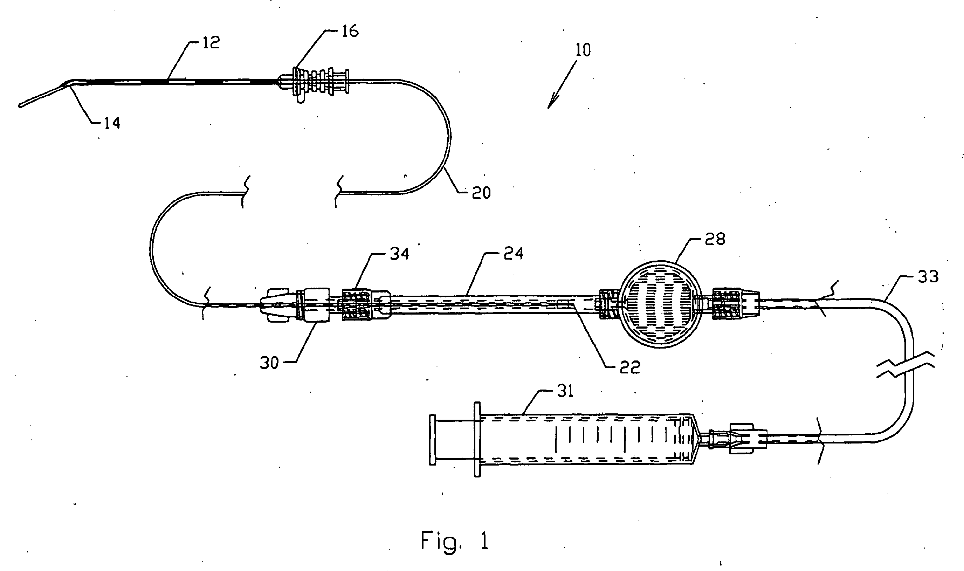

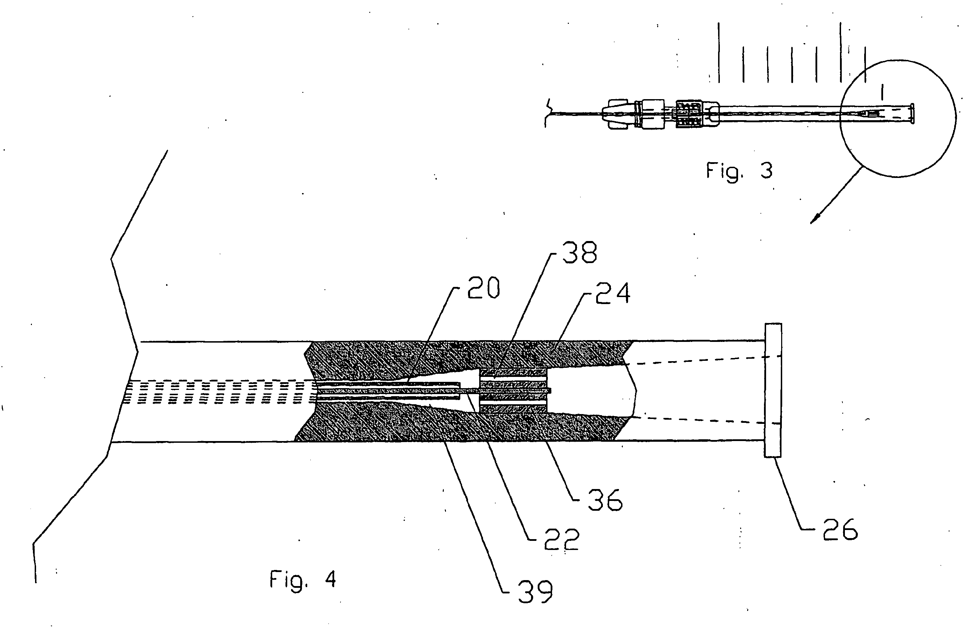

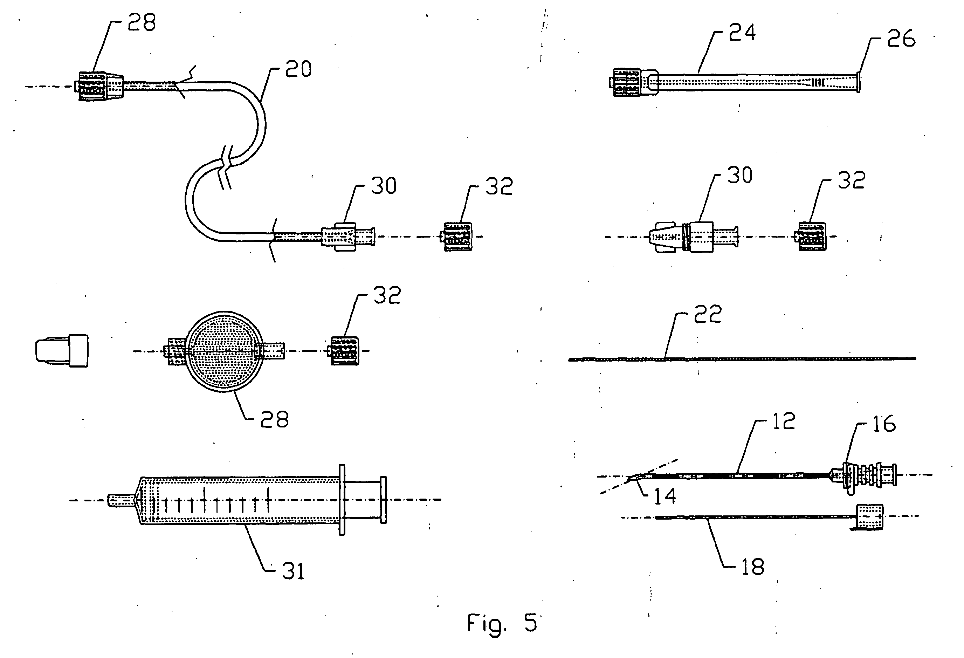

Damit das distale Ende des Katheters 20 eine gewünschte Steifigkeit erfährt, wird das Führungselement 22 in Form eines Drahtes oder Rohres relativ zu dem Katheter 20 fixiert, wobei dessen distales Ende zum distalen Ende des Katheters 20 beabstandet sein kann, insbesondere dann, wenn der Katheter 20 gelegt ist. Damit das Führungselement 22 eine eindeutige Position zu dem Katheter 20 einnimmt, wodurch erwähntermaßen die Flexibilität des distalen Endes des Katheters 20 vorgegeben wird, geht das Führungselement 22 von der Halterung 24 aus und ist nach dem Ausführungsbeispiel der Fig. 1 bis 5 in dessen kanülenfernliegenden Ende über ein zylinderisches Halteelement 36 fixiert. Damit ungeachtet dessen die Halterung 24 von dem über das Filterelement 28 zuzuführenden Fluid durchströmbar ist, weist das Halteelement 36 Durchlässe 38 auf, die auf einen konzentrisch das Halteelement 22 umgebenden Ring bzw. Zylinder angeordnet sein können.

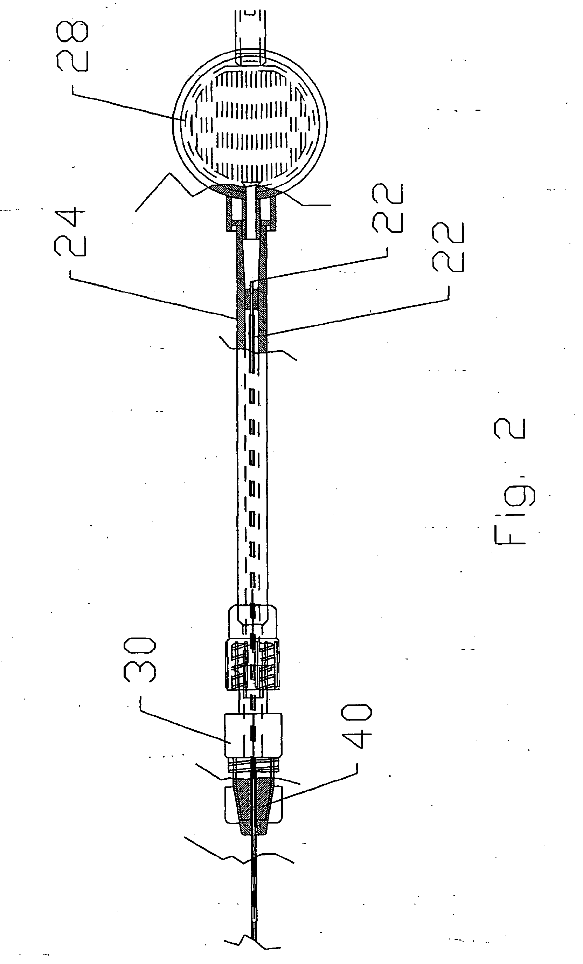

Im Innenraum 39 der Halterung 24 ist der Katheter 20 entlang des Führungselementes 22 verschiebbar. Hierdurch wird im gewünschten Umfang der Abstand zwischen dem distalen Ende des Führungselementes 22 und dem des Katheters 20 zur Einstellung dessen Flexibilität verändert. Sobald der Katheter 20 bzw. dessen distales Ende eine gewünschte Position einnimmt, wird der Katheter 20 über den Tuohy-Burst-Adapter 30 oder ein gleichwirkendes Element fixiert. Hierzu durchsetzt der Katheter 20 ein im Schnitt kegelstumpfförmiges Gummielement und wird mittels des Tuohy-Burst-Adapters 30 in diesem festgequetscht, wie die Prinzipdarstellung der Fig. 2 verdeutlicht.

Demzufolge verbleibt das Führungselement 22 auch bei gelegtem Katheter 20 in diesem. so dass während der medizinischen Applikation der Katheter 20 stets die gewünschte Stei- ' figkeit aufweist. Auch erwächst nicht die nach dem Stand der Technik bestehende Gefahr, dass eine ungewollte Lageveränderung des Katheters durch Entfernen des Führungsetementes erfolgt.

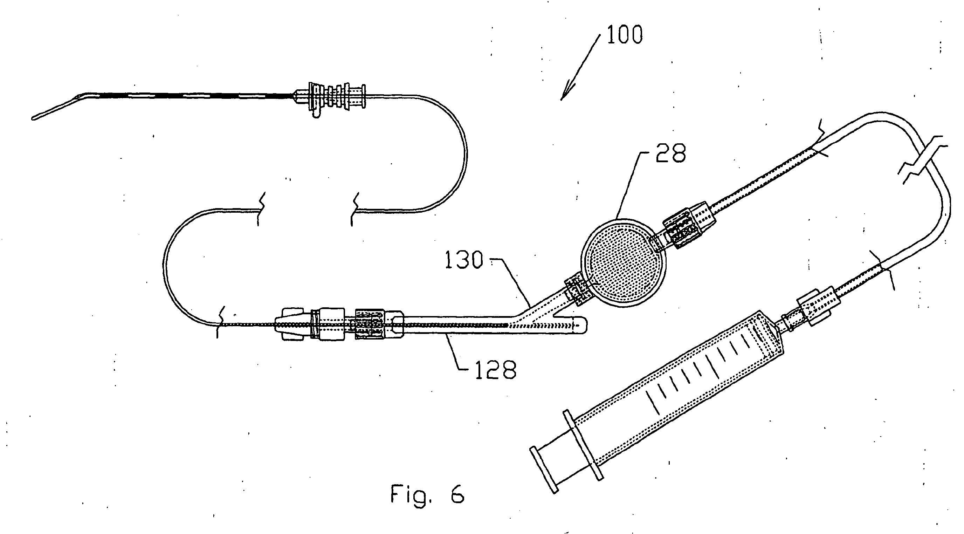

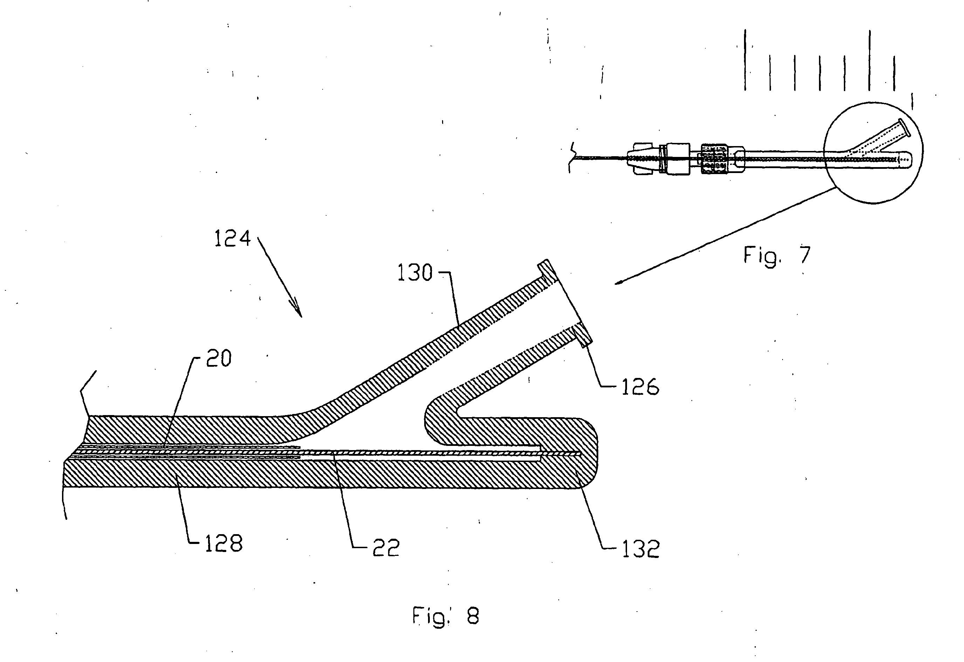

Ist nach dem Auführungsbeispiel der Fig. 1 bis 5 die das Führungselement 22 aufnehmende Halterung, 24 als langgestrecktes Rohrelement ausgebildet, so weist die Halterung 124 nach dem Ausführungsbeispiel der Fig. 6 bis 8 eine Y-förmige Geometrie auf, die sich aus einem entlang einer Gerade verlaufenden ersten Schenkel 128 und einem geneigt zu diesem und in diesen übergehenden zweiten Schenkel 130 zusammensetzt. Dabei geht vom freien Ende des zweiten Schenkels 136 das Filterelement 28 aus.

In dem ersten Schenkel 128, und zwar in dessen kanülenfernliegendem Ende 132 ist das Führungselement 22 festgelegt, Innerhalb der zentralen Bohrung des ersten Schenkels 128 ist sodann relativ zu dem Führungselement 22 der Kitheler 20 in zuvor beschriebener Weise verschiebbar, um in gewünschter Position fixiert zu werden.

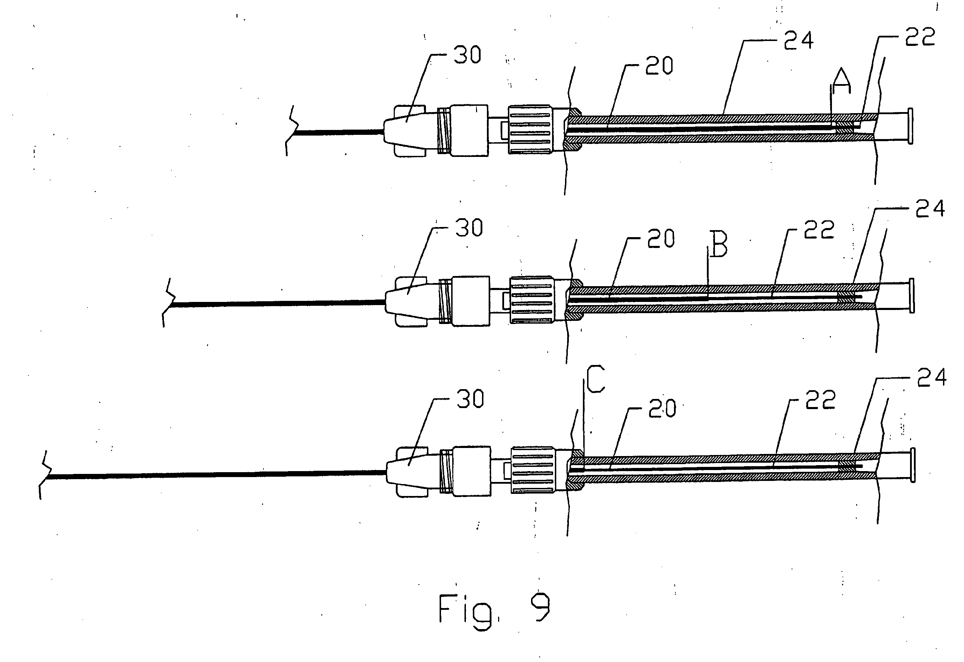

anhand der Fig. 9 ist erkennbar, dass das Führungselement 22 in Form von zum Beispiel einem Rohr aus einer Form-Gedächtnis-Legierung wie Nickel-Titan-Leoierung zu dem Katheter 20 derart ausgerichtet ist, dass die freien Ende des Führungselementes 22 und des Katheters 20 in gewünschtem Abstand enden oder übereinstimmen. So soll die obere Darstellung in Fig. 9 eine Position andeuten, in der die distalen Enden vom Katheter 20 und Fürungselement 22 gleich enden. Diese auch als erste Position zu bezeichnende Zuordnung kann beim Legen des Katheters 20 vorgegeben werden.

Demgegenüber ist bei der minieren Darstellung in Fig. 9 das Führungselement 22 scheinbar zurückgezogen, so dass der Katheter 20 am distalen Ende flexibel ist. Eine noch höhere Flexibilität des distalen Endes des Katheters 20 ergibt sich bei einer Ausrichtung von Führungselement 22 zum Katheter 20 in der unteren Darstellung der Fig. 9.

Entsprechend der Abstände zwischen den Markierungen A. B und C in der Fig. 9 verlauft das Führungselement 22 mit seinem distalen Ende beabstandet zum distalen Ende des Katheters 20, also innerhalb des Katheters 20, wodurch erwähntermaßen eine gezielte Flexibilität vorgegeben werden kann. wobei die beiden unteren Darstellungen einer zweiten Position bei einem in einem Epiduralraum gelegten Katheter 20 entsprechen können.

1st im Ausführungsbeispiel der Fig. 9 das Führungselement 22 als Rohr bezeichnet worden, so ist die Verwendung eines Drahtes, der ebenfalls aus einer Nickel-Titan-Legierung bestehen kann, gleichfalls möglich.

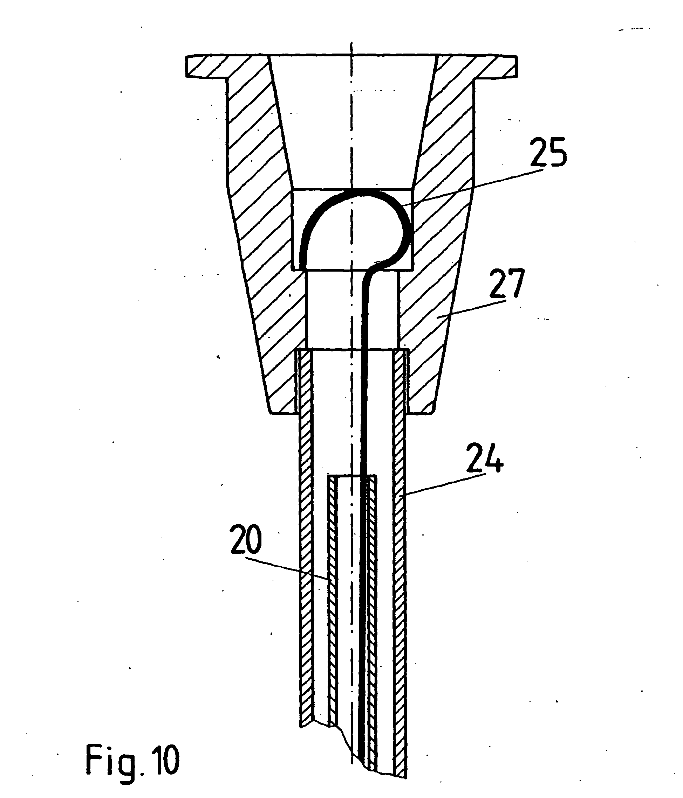

Der Fig. 10 ist eine weitere Lösungsmöglichkeit zum Fixieren des Führungselementes 22 zu entnehmen. So kann dieses, das heißt ein U- oder bogenförmiger gebogener Endabschnitt 25 im proximalen Bereich der Halterung, insbesondere in einer Erweiterung 27 dieser durch Klemmen fixiert werden. Dabei kann die Erweiterung 27 als Aufsatz auf ein oder das Rohrelement 24 ausgebildet sein, in dem der Katheter 20 verschiebbar ist.

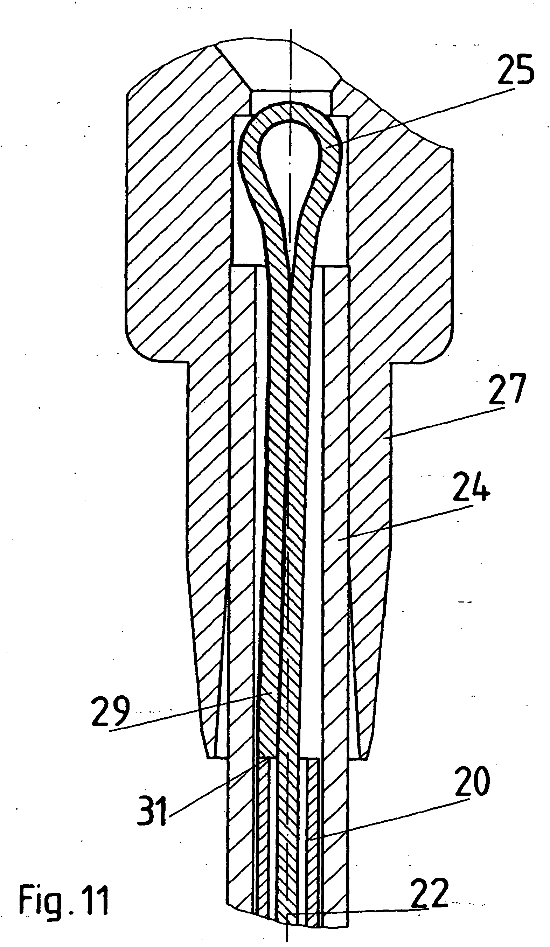

Die Fig. 11 stellt eine alternative Ausführungsform zu der der Fig. 10 zu entnehmenden insoweit dar, als dass der U-förmig gebogene Endabschnitt 25 des Führungselementes 22 Anschlag für den Katheter 20 bildet. So erstreckt sich das Führungselement 22 mit seinem - freien Endabschnitt 29 innerhalb des in Fig. 11 prinzipiell dargestellten Rohrabschnitts 24, so dass freie Stirnfläche 31 des Endabschnitts 25 Anschlag für den Katheter 20 bildet.

Bevorzugte Dimensionierungen von Katheter 20 und Führungselement 22 in Form eines Rohres und Drahtes sind:

| | | |
|---|---|---|
| Außendurchmesser des Katheters 20: | | 0,8 mm bzw. 2.3 mm |
| | | |
| Durchmesser eines Drahtes als Führungselement 22: | | zwischen 0.30 und 0,55 mm. |
| | | |
| Dimensionierung eines Rohres als. Führungselement 22: | Außendurchmesser: | 0,4 mm - 0,6 mm |
| | Wandstärke: | 0,1 mm. |

Der Katheter 20 selbst ist vorzugsweise ein Schlauchelement das aus Polyamid besteht.

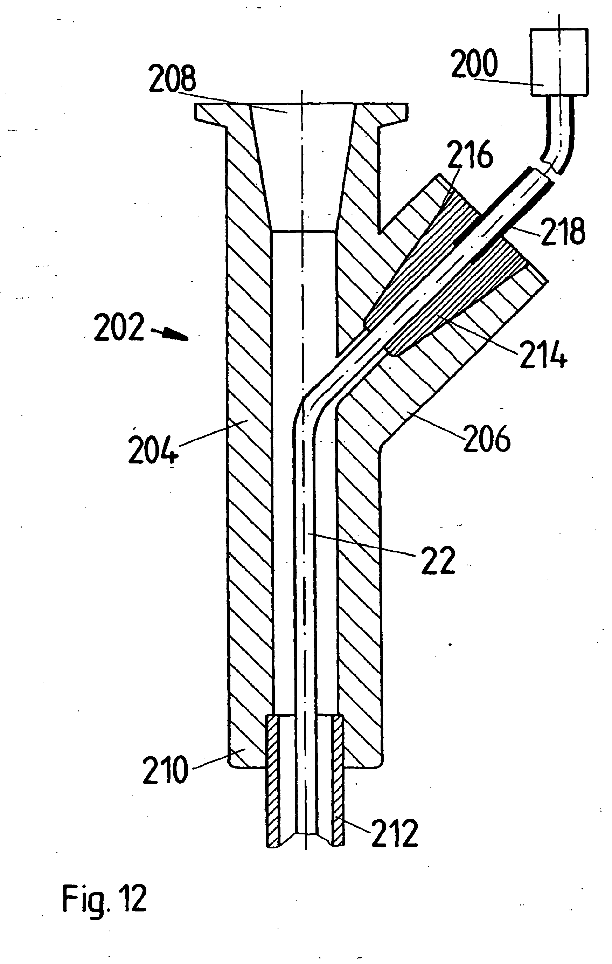

Den Fig. 12 bis 14 sind weitere bevorzugte Ausgestaltungen der erfindungsgemäßen Lehre zu entnehmen, soweit das Führungselement 22 in Form eines elektrisch leitenden Elementes wie Drahtes betroffen ist. So kann die erfindungsgemäße Verweilkatheteranordnung auch zur Elektrostimulation von Gewebebereichen insbesondere im Epiduralbereich benutzt werden. Hierzu wird das Führungselement 22 über einen Anschluss 200 mit eine Pol einer Spannungsquelle verbunden, um so als monopolar ausgebildete Elektrode zu wirken. Um gleichzeitig die Möglichkeit zu bieten. über den Katheter 20, innerhalb der das Führunselement 22, zum Einstellen der Steifigkeit des Katheters 20 verläuft. Medikamente zu applizieren, ist eine Halterung 202 vorgesehen, die im Ausführungsbeispiel eine Y-Geometrie mit einem langgestreckten ersten Schenkel 204 und einem zu diesem geneigt verlaufenden zweiten Schenkel 206 aufweist. Der langgestreckte Schenkels 204 weist endseitig eine Öffnung 208 auf über die in zuvor beschriebener Weise gegebenfalls über einen Filter ein Medikament zugeführt werden kann. Das der Öffnung 208 gegenüberliegende Ende 210 ist sodann mit einem Rohrelement 212 verbunden, entlang der das Führungselement 22 verläuft und zu dem der nicht dargestellte Katheter im gewünschten Umfang verschiebbar ist. Insoweit wird jedoch auf die Erläuterungen zu den Ausführungsbeispielen der Fig. 1 bis 9, insbesondere der Fig. 9 verwiesen. Über den Zwischenraum von Katheter 20 und Führungselement 22 gelangt sodann das Medikament zum distalen Ende des Katheters 20, um bei geschlossenem Katheter 20 aus im distalen Bereich vorhandene Öffnungen oder bei nicht geschlossenem Katheter 20 über dessen Spitze austreten zu können.

Das Führungselement 22 durchsetzt den abgewinkelten Schenkel 206 der Halterung 202 und ist von einem Stoptenelement 214 umgeben, das flüssigkeitsdicht in endseitiger Öffnung 216 des zweiten Schenkels 206 eingesetzt ist. Außerhalb der Halterung 202 bzw. des Verschlusselementes 214 ist das führungselement 22 von einer elektrischen Isolierung 218 umgeben, um sodann in den Anschluss 200 überzugehen, der mit einer Spannungsquelle verbindbar ist.

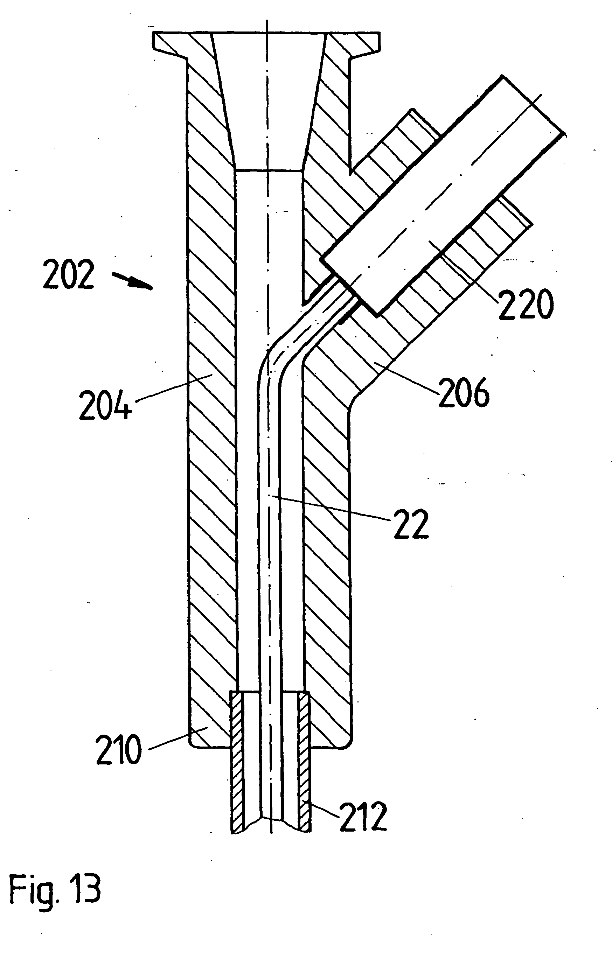

Bei dem Ausführungsbeispiel der Fig. 13 in dem der Fig. 12 entsprechende Elemente mit gleichen Bezugszeichen versehen sind, geht das Führungselement 22 in ein Anschlusselement 220 wie Metallbuchse über, die ihrerseits flüssigkeitsdicht in den zweiten Schenkel 206 fixiert ist. Von dem Anschlusselement 220 verläuft sodann eine Verbindung zu einer Spannungsquelle.

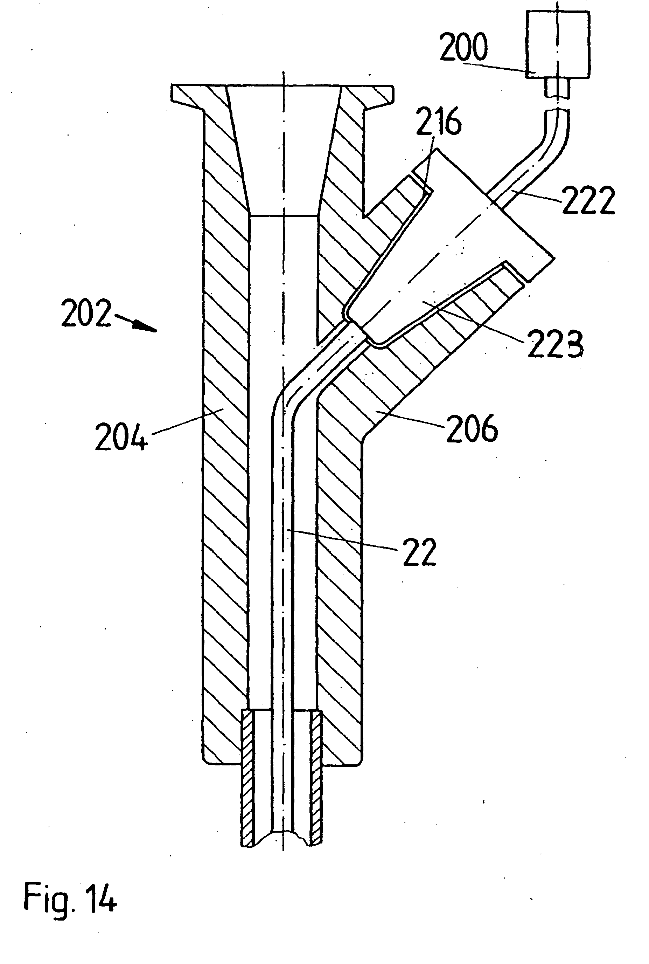

Nach einer besonderen Ausgestaltung gemäß Fig. 14 ist dass Führungselement 22 in seinem proximalen Ende mit einem elektrischen Leiter 222 verbunden, wobei der Verbindungsbereich mit Kunststoff umspritzt ist. Dabei wird beim Spritzen eine Geometrie gebildet, die eine Stopfenform aufweist. Ein entsprechender Stopfen 223 ist sodann in der Öffnung 216 des zweiten Schenkels 206 der Halterung 202 flüssigkeitsdicht eingesetzt wie eingeklebt.

Durch die entsprechenden den Fig. 12 bis 14 zu entnehmenden Maßnahmen ist eine sichere Verbindung zwischen dem Führungselement 22 und einem elektrischen Anschluss möglich, wobei der flüssigkeitsdichte Abschluss nach außen erfolgt. Gleichzeitig wird das Fühmngselement 22 durch den Stopfen 214, 223 oder die Metallbuchse 220 in der Halterung 202 fixiert, wodurch die erwünschte Unverrückbarkeit und somit die Möglichkeit gegeben ist, den in den Fig. 12 bis 14 nicht dargestellien Katheter in gewünschtem Umfang zu dem Katheter 22 zu verschieben, um einerseits die Steifigkeit des Katheters 20 in seinem distalen Bereich einsteilen zu können und andererseits über das distale Ende des Führungselementes 22 den Katheter 20 derart ausrichten zu können, dass, wenn das Führungselement 22 als Elektrode wirkt, im erforderlichen Umfang eine Elektrostimulation erfolgen kann.

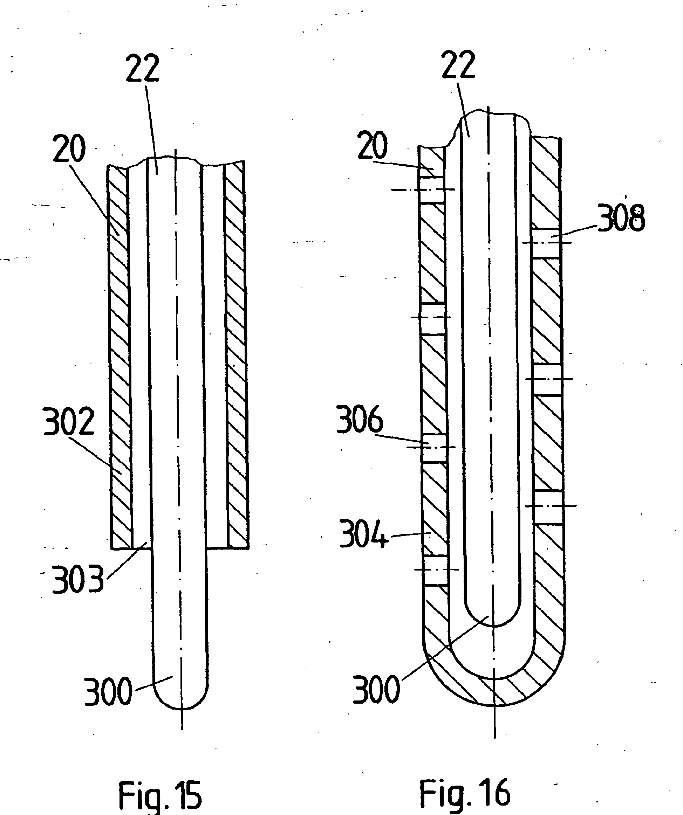

Damit im erforderlichen Umfang ein Stromfluss zwischen dem Fuhrungselement 22, d. h. seinem distalen Ende 300 und dem zu stimulierenden Gewebe erfolgen kann, sind entsprechend der Darstellungen in Fig. 15 und 16 verschiedene Möglichkeiten gegeben. So kann der distale Bereich 302 des Katheters 20 offen sein, so dass bei der Elektrostimulation das distale Ende 300 des Führungselements 22 den Katheter 20 durchsetzt (Fig. 15).

Alternativ kann der Katheter 20 distal verschlossen sein, wie dies auch bei den Ausführungsbeispielen der Fig. 1 bis 10 möglich ist. Um Medikamente zu applizieren, ist es sodann nur erforderlich, ein oder zwei Öffnungen im distalen Bereich des endseitig geschlossenen Katheters 22 vorzusehen. Im Falle einer Elektrostimulation sind jedoch grundsätzlich mehr Öffnungen erforderlich, wie dies aus der Fig. 16 ersichtlich ist. So weist im Ausführungsbeispiel der Fig. 16 der Katheter 22 in seinem distalen Bereich 304 mehrere Öffnungen auf, die beispielhaft mit den Bezugszeichen 306 und 308 versehen sind. Insbesondere sind 8 bis 12 Öffnungen 306, 308 vorgesehen, im Bereich derer das Führungselement 22 mit seinem distalen Bereich 300 bei gelegtem Katheter verläuft.

## Patentansprüche

1. Verweilkatheteranordnung (10, 100) umfassend eine Kanüle (12), deren proximales Ende von einer Aufnahme (16) ausgeht und deren distales Ende eine Öffnung (14), insbesondere einen Tuohyschliff mit seitlicher Öffnung aufweist, sowie einen durch die Aufnahme und durch die Öffnung der Kanüle führbaren flexiblen Katheter (20), in dem seinerseits beim Legen des Katheters ein Führungselement (22) verläuft, wobei die Verweilkatheteranordnung eine Halterung aufweist und der Katheter mit seinem proximalen Ende verschiebbar in der Halterung verläuft,
**dadurch gekennzeichnet,**
**dass** das Führungselement (22) in der Halterung (24, 124) fixierbar ist und dass das nach dem Legen des Katheters (20) in diesem verbleibende Führungselement in der Halterung relativ zu dem Katheter in definierter Position fixiert ist.

2. Verweilkatheteranordnung nach Anspruch 1
**dadurch gekennzeichnet,**
**dass** das Führunselement (22) wie Draht oder Rohr während des Legens des Katheters (20) zu diesem in einer ersten Position und bei gelegtem Katheter zu diesem in einer zweiten Position relativ fixiert ist.

3. Verweilkatheteranordnung nach Anspruch 1 ,
**dadurch gekennzeichnet.**
**dass** die Halterung (24,124) bereichsweise rohrförmig ist.

4. Verweilkatheteranordnung nach Anspruch 1
**dadurch gekennzeichnet,**
das der Katheter (20) über ein Klemmelement (40) fixiert ist.

5. Verweikkatheteranordnung nach zumindest einem der vorhergehenden Ansprüche.
**dadurch gekennzeichnet.**
**dass** der Katheter (20) mittels eines Tuob-Burst-Adapters (30) oder gleichwirkendes Element fixiert ist.

6. Verweilkatheteranordnung nach Anspruch 1
**dadurch gekennzeichnet.**
**dass** die Halterung (24, 124) einen Fluidanschluss (26, 126) aufweist, dem ein Filter (28) vorschaltbar ist.

7. Verweilkatheteranordnung nach Anspruch 1,
**dadurch gekennzeichnet.**
das die Halterung (124) eine Y- oder V-förmige Geometrie aufweist mit einem entlang einer Gerade verlaufenden ersten Schenkel (128) und einem zu diesem geneigt verlaufenden und in diesen übergehenden zweiten Schenkel (130) und dass in dem ersten Schenkel das Führungselement (22) fixiert und in diesem das Führungselement umgebend das proximale Ende des Katheters (20) verschiebbar angeordnet ist.

8. Verweilkatheteranordung nach zumindest einem der vorhergeilenden Ansprüche,
**dadurch gekennzeichnet,**
**dass** das Führungselement (22) in der Halterung (24) von einem Halteelement (36) gehalten ist, das seinerseits zumindest einen Fluiddurchlass (38) aufweist.

9. Verweilkatheteranordnung nach zumindest Anspruch 8
**dadurch gekennzeichnet,**
**dass** der Fluiddurchlass (38) durch das Führungselement (22) konzentrisch umgebende Durchlässe (28) gebildet ist.

10. Verweilkatheteranordnung nach zumindest Anspruch 1
**dadurch gekennzeichnet,**
**dass** der Katheter (20) über eine Quetschfixierung festgelegt ist.

11. Verweilkatheteranordung nach zumindest Anspruch 1
**dadurch gekennzeichnet.**
**dass** das Führungselement (22) in der Halterung (24, 124) durch insbesondere Kleben fixiert ist.

12. Verweilkatheteranordung nach zumindest Anspruch 1
**dadurch gekennzeichnet.**
**dass** das Führungselement (22) in einer zur Kanüle (12) fernliegenden Wandung (132) der Halterung (124) fixiert ist.

13. verweilkatheteranordung nach zumindest Anspruch 1,
**dadurch gekennzeichnet,**
**dass** das Führungselement in einem proximalen Bereich der Halterung durch Klemmen, insbesondere durch Klemmen eines gebogenen Endabschnitts des Führungselementes fixiert ist.

14. Venweilkatheteranordnung nach zumindest einem der vorhergehenden Ansprüche,
**dadurch gekennzeichnet.**
**dass** das Führungselement (22) aus einer Form-Gedächtnis-legiening wie Nickel-Titan-Legierung besteht oder diese enthält

15. Verweilkatheteranordung noch zumindest einem der vorhergehenden Ansprüche,
**dadurch gekennzeichnet.**
**dass** das Führungselement (22) in Form eines Drahtes einen Durchmesser zwischen 0.30 mm und 0.55 mm aufweist.

16. Verweilkatheteranordung nach zumindest einem der vorhergehenden Ansprüche,
**dadurch gekennzeichnet,**
**dass** der Katheter (20) einen Außendurchmesser zwischen 0,8 mm und 2,3 mm ausweist.

17. Verweilkatheteranordnung nach zumindest einem der vorhergehenden Ansprüche.
**dadurch gekennzeichnet.**
**dass** das Führungselement (22) ein Rohrelement ist, dessen Außendurchmesser in etwa 0,4 mm bis 0.6 mm und dessen Wandstärke in etwa 0,1 mm betragen.

18. Verweilkatheteranordnung nach zumindest einem der vorhergehenden Ansprüche.
**dadurch gekennzeichnet.**
**dass** der Katheter (20) vorzugsweise aus Polyamid, Polymethan oder Silicon besteht.

19. Verweilkatheteranordnung nach zumindest Anspruch 1
**dadurch gekennzeichnet.**
**dass** die Kanüle eine Spalikanüle ist.

20. Verweilkatheteranordnung nach Anspruch 1
**dadurch gekennzeichnet,**
**dass** das Führungselement (22) zum Fixieren endseitig einen U-förmigen Verlauf zeigt, wobei freie Stirmfläche (31) umgebogenen Abschnitts (29) des Führungselementes proximaler Anschlag für den zu dem Führungselement verschiebbaren Katheter (20) ist.

21. Verweilkatheteranordnung nach zumindest Anspruch 1.
**dadurch gekennzeichnet,**
**dass** das Führungselement (22) eine mit einer Spannungsquelle verbindbare Elektrode ist, die im distalen Bereich (302, 304) des Katheters (20) über zumindest eine Öffnung (303, 306, 308) in elektrisch leitenden Kontakt mit einem Gewebe bzw. einer Gewebeflüssigkeit bringbar ist.

22. Verweilkatheteranordnung nach zumindest einem der vorhergehenden Ansprüche,
**dadurch gekennzeichnet.**
**dass** das Führungselement (22) den Katheter (20) in seinem distalen Ende (302) durchsetzt.

23. Verweilkatheteranordnung nach zumindest einem der vorhergehenden Ansprüche,
**dadurch gekennzeichnet.**
**dass** distaler Bereich (300) des Führungselementes (22) stets innerhalb des Katheters (22) verläuft.

24. Verweilkatheteranordnung nach zumindest Anspruch 1
**dadurch gekennzeichnet.**
**dass** das Führungselement (22) in einer Halterung (202) mit vorzugsweise V- oder Y-artiger Geometrie fixiert ist, dass die Halterung (200) einen geradlinig verlaufenden ersten Schenkel 204 aufweist, entlang dem das Führungselement (22) und der diesen umgebenden und zu diesem verschiebbare Katheter (20) abschnittsweise oder in dessen Richtung verlaufen, und dass die Halterung einen geneigt zu dem ersten Schenkel verlaufenden zweiten Schenket (206) umfasst, in dem das Führungselement fixiert ist.

25. Verweilkatheteranordnung nach Anspruch 7 oder 24,
**dadurch gekennzeichnet,**
**dass** das Führungselement (22) in der Halterung (202) bzw. dessen zweiten Schenkel (206) über einen in einer Öffnung (216) flüssigkeitsdicht eingesetzten Stopfen (214, 222) unverrückbar fixiert ist.

26. Verweilkaltheteranordnung nach Anspruch 25
**dadurch gekennzeichnet,**
**dass** der Stopfen (214. 222) in der Öffnung (216) des zweiten Schenkels (206) flüssigkeitsdicht fixiert wie eingeklebt ist.

27. Verweilkatheteranordnung nach zumindest Anspruch 7 oder 24
**dadurch gekennzeichnet,**
**dass** das führungselement (22) von einer Metallbuchse (220) ausgeht, die flüssigkeirsdicht in dem zweiten Schenkel (206) bzw. dessen Öffnung (216) eingesetzt ist.

28. Verweilkatheteranordnung nach zumindest Anspruch 7 oder 24.
**dadurch gekennzeichnet.**
**dass** über den ersten Schenkel (204) der Halterung (202) Medikamente zuführbar sind.

## Claims

1. Indwelling catheter arrangement (10, 100) comprising a canula (12)whose proximal end extends from a receptacle (16) and whose distal end has an opening, in particular, a Tuohy's cut with lateral opening, as well as a flexible catheter (20) that can be led through the receptacle and through the opening of the canula, in which in turn a guiding element (22) runs during the positioning of the catheter, whereby the indwelling catheter arrangement comprises a bracket and the catheter runs with its proximal end displaceable in the bracket,
**characterized in**
**that** the guiding element (22) can be fixed in the bracket (24, 124) and that the guiding element remaining in the catheter after the positioning of the catheter (20) is fixed in a defined position relative to the catheter.

2. Indwelling catheter arrangement according to claim 1,
**characterized in**
**that** the guiding element (22) such as a wire or a pipe is fixed during the positioning of the catheter (20) relative to it in a first position and with positioned the catheter relative to it in a second position.

3. Indwelling catheter arrangement according to claim 1,
**characterized in**
**that** the bracket (24, 124) is tubular in sections.

4. Indwelling catheter arrangement according to claim 1,
**characterized in**
**that** the catheter (20) is fixed via a clamping element (40).

5. Indwelling catheter arrangement according to at least one of the previous claims,
**characterized in**
**that** the catheter (20) is fixed via a Tuohy burst adapter (30) or an equally acting element.

6. Indwelling catheter arrangement according to claim 1,
**characterized in**
**that** the bracket (24, 124) has a fluid connection (26, 126), upstream of which a filter (28) can be connected.

7. Indwelling catheter arrangement according to claim 1,
**characterized in**
**that** the bracket (124) has a Y- or V-shaped geometry with a first leg (128) running along a straight line and a second leg (130) running inclined to the first leg and merging with it and that in the first leg the guiding element (22) is fixed and that in it the proximal end of the catheter (20) surrounding the guiding element is displaceable arranged.

8. Indwelling catheter arrangement according to at least one of the previous claims,
**characterized in**
**that** the guiding element (22) is held in the bracket (24) by a holding element (36), which, in turn, has at least one fluid passage (38).

9. Indwelling catheter arrangement according to at least claim 8,
**characterized in**
**that** the fluid passage (38) is formed by passages (28) surrounding the guiding element (22) concentrically.

10. Indwelling catheter arrangement according to at least claim 1,
**characterized in**
**that** the catheter (20) is fixed by a squeeze fixation.

11. Indwelling catheter arrangement according to at least claim 1,
**characterized in**
**that** the guiding element (22) is fixed in the clamping element (24, 124) by in particular glueing.

12. Indwelling catheter arrangement according to at least claim 1,
**characterized in**
**that** the guiding element (22) is fixed in a wall (132) of the bracket (124) remote to the canula (12).

13. Indwelling catheter arrangement according to at least claim 1,
**characterized in**
**that** the guiding element is fixed in a proximal area of the bracket through clamps, in particular through clamps of a bent end segment of the guiding element.

14. Indwelling catheter arrangement according to at least one of the previous claims,
**characterized in**
**that** the guiding element (22) consists of or contains a shape memory alloy such as a nickel-titanium alloy.

15. Indwelling catheter arrangement according to at least one of the previous claims,
**characterized in**
**that** the guiding element (22) in form of a wire has a diameter ranging from 0.30 mm to 0.55 mm.

16. Indwelling catheter arrangement according to at least one of the previous claims,
**characterized in**
**that** the catheter (20) has an outer diameter between 0.8 mm and 2.3 mm.

17. Indwelling catheter arrangement according to at least one of the previous claims,
**characterized in**
**that** the guiding element (22) is a pipe element whose outer diameter is between approximately 0.4 mm and 0.6 mm and whose wall thickness is approximately 0.1 mm.

18. Indwelling catheter arrangement according to at least one of the previous claims,
**characterized in**
**that** the catheter (20) consists preferably of polyamide, polymethane or silicon.

19. Indwelling catheter arrangement according to at least claim 1,
**characterized in**
**that** the canula is a split canula.

20. Indwelling catheter arrangement according to claim 1,
**characterized in**
**that** for fixation the guiding element (22) exhibits a U-shaped course at the end side, with free front surface (31) of bent segment (29) of the guiding element being proximal stop for the catheter (20) displaceable relative to the guiding element.

21. Indwelling catheter arrangement according to at least claim 1,
**characterized in**
**that** the guiding element (22) is an electrode that can be connected to a power source, which can be brought into electrically conductive contact in the distal area (302, 304) of the catheter (20) via at least one opening (303, 306, 308) with a tissue and with a tissue fluid, respectively.

22. Indwelling catheter arrangement according to at least one of the previous claims,
**characterized in**
**that** the guiding element (22) passes through the catheter (20) at its distal end (302).

23. Indwelling catheter arrangement according to at least one of the previous claims,
**characterized in**
**that** distal area (300) of the guiding element (22) runs always within the catheter (22).

24. Indwelling catheter arrangement according to at least claim 1,
**characterized in**
**that** the guiding element (22) is fixed in a bracket (202) with a preferably V- or Y-like geometry, that the bracket (200) has a linearly running first leg (104) along which the guiding element (22) and the catheter (20), surrounding said element and being displaceable thereto, run in sections or in its direction, and that the bracket comprises a second leg (206) running inclined to the first leg, in which the guiding element is fixed.

25. Indwelling catheter arrangement according to claim 7 or 24,
**characterized in**
**that** the guiding element (22) is immovably fixed in the bracket (202) and in its second leg (206), respectively, via a plug (214, 222) that is inserted fluid-proof in an opening (216).

26. Indwelling catheter arrangement according to claim 25,
**characterized in**
**that** the plug (214, 222) is fixed fluid-tight, such as glued, into the opening (216) of the second leg (206).

27. Indwelling catheter arrangement according to at least claim 7 or 24,
**characterized in**
**that** the guiding element (22) extends from a metal bushing (220) that is inserted into the second leg (206) or its opening (216) in a fluid-tight manner.

28. Indwelling catheter arrangement according to at least claim 7 or 24,
**characterized in**
**that** medication can be administered via the first leg (204) of the bracket (202).

## Revendications

1. Système de cathéter à demeure (10, 100) comprenant une canule (12) dont l'extrémité proximale part d'un logement (16) et dont l'extrémité distale présente un orifice (14), en particulier un biseau Tuohy avec orifice latéral, ainsi qu'un cathéter souple (20) pouvant être introduit à travers le logement et l'orifice de la canule, dans lequel passe un élément de guidage (22) lors de la pose du cathéter, sachant que système de cathéter présente un raccord et qu'avec son extrémité proximale, le cathéter passe dans le raccord de manière à pouvoir être déplacé,
**caractérisé en ce**
**que** l'élément de guidage (22) peut être fixé dans le raccord (24, 124), et que l'élément de guidage qui reste dans le raccord après la pose du cathéter (20) est fixé dans le raccord dans une position définie par rapport au cathéter.

2. Système de cathéter à demeure selon la revendication 1,
**caractérisé en ce**
**que** lors de la pose du cathéter (20), l'élément de guidage (22) tel qu'une fil métallique ou un tube est fixé dans une première position par rapport à celui-ci, et qu'une fois le cathéter posé, l'élément de guidage est fixé dans une seconde position par rapport à celui-ci.

3. Système de cathéter à demeure selon la revendication 1,
**caractérisé en ce**
**que** le raccord (24, 124) a sectoriellement une forme tubulaire.

4. Système de cathéter à demeure selon la revendication 1,
**caractérisé en ce**
**que** le cathéter (20) est fixé par un élément de serrage (40).

5. Système de cathéter à demeure selon au moins l'une des revendications précédentes,
**caractérisé en ce**
**que** le cathéter (20) est fixé par un adaptateur Tuohy-Burst (30) ou par un élément ayant la même fonction.

6. Système de cathéter à demeure selon la revendication 1,
**caractérisé en ce**
le raccord (24, 124) présente un embout pour fluides (26, 126) en amont duquel peut être placé un filtre (28).

7. Système de cathéter à demeure selon la revendication 1,
**caractérisé en ce**
**que** le raccord (124) présente une géométrie en forme de Y ou de V avec une première branche (128) s'étendant le long d'une droite, et une seconde (130) branche inclinée par rapport à la première et se fondant en elle, et que dans la première branche est fixé l'élément de guidage (22), et que dans celle-ci est placée de manière mobile l'extrémité proximale du cathéter (20) entourant l'élément de guidage.

8. Système de cathéter à demeure selon au moins l'une des revendications précédentes,
**caractérisé en ce**
**que** l'élément de guidage (22) dans le raccord (24) est maintenu par élément de maintien (36) qui présente lui-même au moins un passage pour fluides (38).

9. Système de cathéter à demeure selon au moins la revendication 8,
**caractérisé en ce**
**que** le passage pour fluides (38) est formé par des passages (28) entourant concentriquement l'élément de guidage (22).

10. Système de cathéter à demeure selon au moins la revendication 1,
**caractérisé en ce**
**que** le cathéter (20) est fixé par une fixation par pincement.

11. Système de cathéter à demeure selon au moins la revendication 1,
**caractérisé en ce**
**que** l'élément de guidage (22) est fixé en particulier par collage dans le raccord (24, 124).

12. Système de cathéter à demeure selon au moins la revendication 1,
**caractérisé en ce**
**que** l'élément de guidage (22) est fixé dans un paroi (132) du raccord (124) éloignée de la canule (12).

13. Système de cathéter à demeure selon au moins la revendication 1,
**caractérisé en ce**
**que** l'élément de guidage est fixé par serrage dans une zone proximale du raccord, en particulier par serrage d'une section terminale cintrée de l'élément de guidage.

14. Système de cathéter à demeure selon au moins l'une des revendications précédentes,
**caractérisé en ce**
**que** l'élément de guidage (22) est constitué d'un alliage à mémoire de forme, tel qu'un alliage nickel-titane, ou en contient.

15. Système de cathéter à demeure selon au moins l'une des revendications précédentes,
**caractérisé en ce**
**que** l'élément de guidage (22) sous forme d'un fil métallique présente un diamètre compris entre 0,30 mm et 0,55 mm.

16. Système de cathéter à demeure selon au moins l'une des revendications précédentes,
**caractérisé en ce**
**que** le cathéter (20) présente un diamètre extérieur compris entre 0,8 mm et 2,3 mm.

17. Système de cathéter à demeure selon au moins l'une des revendications précédentes
**caractérisé en ce**
**que** l'élément de guidage (22) un élément tubulaire ayant un diamètre extérieur compris approximativement entre 0,4 mm et 0,6 mm et une épaisseur de paroi approximativement égale à 0,1 mm.

18. Système de cathéter à demeure selon au moins l'une des revendications précédentes,
**caractérisé en ce**
**que** le cathéter (20) est de préférence en polyamide, polyméthane ou silicone.

19. Système de cathéter à demeure selon au moins la revendication 1,
**caractérisé en ce**
**que** la canule est une canule clivable.

20. Système de cathéter à demeure selon la revendication 1,
**caractérisé en ce**
**que** pour la fixation, l'élément de guidage (22) présente une extrémité en forme de U, sachant que la face libre (3) de la section repliée (29) de l'élément de guidage est la butée proximale pour le cathéter (20) mobile par rapport à l'élément de guidage.

21. Système de cathéter à demeure selon au moins la revendication 1,
**caractérisé en ce**
**que** l'élément de guidage (22) est une électrode pouvant être reliée à une source de tension, qui dans la zone distale (302, 304) du cathéter (20), peut être mise en contact conduisant l'électricité avec un tissu ou un liquide tissulaire par au moins un orifice (303, 306, 308).

22. Système de cathéter à demeure selon au moins l'une des revendications précédentes,
**caractérisé en ce**
**que** l'élément de guidage (22) traverse le cathéter (20) dans son extrémité distale (302).

23. Système de cathéter à demeure selon au moins l'une des revendications précédentes,
**caractérisé en ce**
**que** la zone distale (300) de l'élément de guidage (22) s'étend constamment à l'intérieur du cathéter (22).

24. Système de cathéter à demeure selon au moins la revendication 1,
**caractérisé en ce**
**que** l'élément de guidage (22) est fixé dans un raccord (202) présentant de préférence une géométrie en forme de V ou de Y, que le raccord (200) présente une première branche (204) rectiligne le long de laquelle s'étendent sectoriellement ou dans sa direction l'élément de guidage (22) et le cathéter (20) entourant l'élément de guidage et mobile par rapport à lui, et que le raccord comprend une seconde branche (206) inclinée par rapport à la première branche et dans laquelle est fixé l'élément de guidage.

25. Système de cathéter à demeure selon la revendication 7 ou 24,
**caractérisé en ce**
**que** l'élément de guidage (22) est fixé de manière immuable dans le raccord (202) ou dans sa seconde branche (206) par un bouchon (214, 222) enfoncé dans un orifice (216) de manière étanche aux liquides.

26. Système de cathéter à demeure selon la revendication 25,
**caractérisé en ce**
**que** le bouchon (214, 222) est fixé de manière étanche aux liquides, par exemple collé, dans l'orifice (216) de la seconde branche (206).

27. Système de cathéter à demeure selon au moins la revendication 7 ou 24,
**caractérisé en ce**
**que** l'élément de guidage (22) sort d'un manchon métallique (220) inséré de manière étanche aux liquides dans la seconde branche (206) ou dans son orifice (216).

28. Système de cathéter à demeure selon au moins la revendication 7 ou 24,
**caractérisé en ce**
**que** des médicaments peuvent être introduits par la première branche (204) du raccord (202).
